# EUROPÄISCHE PATENTANMELDUNG

(11) **EP 3 628 343 A1**
(43) Veröffentlichungstag der Anmeldung: **01.04.2020**
(21) Anmeldenummer: 18197066.6
(22) Anmeldetag: 27.09.2018
(51) Int. Cl.: A61M 1/06

(54) **BRUSTPUMPE**

(71) Anmelder: Medela Holding AG, 6340 Baar (CH)
(72) Erfinder: MASUCCI, Andrea, 8708 Männedorf (CH)
(74) Vertreter: Clerc, Natalia

(57) **Zusammenfassung**

Eine elektrisch betriebene Brustpumpe zum Abpumpen von menschlicher Muttermilch weist ein elektrisches Pumpaggregat (15) und eine elektronische Steuereinheit (14) zur Steuerung des Pumpaggregats (15) auf. Das Pumpaggregat (15) ist mittels der Steuereinheit (14) zeitverzögert aktivierbar. Die erfindungsgemässe Brustpumpe ermöglicht auf eine einfache Art und Weise einen stressfreien Start beim Abpumpen von Muttermilch.

## Beschreibung

### TECHNISCHES GEBIET

Die vorliegende Erfindung betrifft eine elektrisch betriebene Brustpumpe zum Abpumpen menschlicher Muttermilch.

### STAND DER TECHNIK

Brustpumpen zum Abpumpen von menschlicher Muttermilch sind hinlänglich bekannt. Grundsätzlich gibt es zwei verschiedene Typen: der erste wird manuell betrieben, d.h. der für das Abpumpen notwendige Unterdruck wird durch manuelle Betätigung einer Vakuumpumpe erzeugt. Beim zweiten Typ ist ein elektrisches Pumpaggregat vorhanden mit einem Elektromotor, um den notwendigen Unterdruck zu erzeugen.

Elektrisch betriebene Brustpumpen sind unterschiedlich kompliziert in ihrer Steuerung aufgebaut. Es gibt einfach wirkende Brustpumpen, welche einen gleichbleibenden pulsierenden Unterdruck an die Brustpumpe anlegen, wobei üblicherweise die Pumpfrequenz und die Saugstärke gewählt werden können. Andere Brustpumpen lassen sich mit relativ komplexen Saugkurven programmieren, um ein möglichst naturgetreues Saugverhalten zu erzeugen. Viele dieser Pumpen bieten zudem die Möglichkeit, beide Brüste gleichzeigt abzupumpen. Hierzu müssen zuerst beide Brusthauben dicht auf die Brüste gelegt werden und erst dann beginnt die Brustpumpe optimal zu arbeiten.

Unmittelbar vor dem Abpumpen ist die Mutter somit damit beschäftigt, die Brusthauben optimal auf beiden Brüsten zu positionieren. Sie hat dadurch keine Hand frei, um die Brustpumpe zu aktivieren. Schaltet sie jedoch zuerst die Brustpumpe ein und legt erst dann die Brusthauben auf die Brüste, verhindert der bereits erzeugte Unterdruck ein optimales und für die Mutter schmerzfreies Auflegen der Brusthauben auf die beiden Brüste.

Aus WO 2008/009145 ist eine Brustpumpeneinheit bekannt, bei welcher in der Brusthaube eine Signalsendeeinheit und im Pumpengehäuse eine Empfängereinheit angeordnet ist. Die Empfängereinheit ist mit der Steuerung des Pumpaggregats verbunden. Dadurch kann die Mutter während des Abpumpens die Einstellung der Brustpumpe verändern, d.h. die Pumpfrequenz und die Saugstärke, d.h. den angelegten Unterdruck. Trotzdem muss die Mutter bereit und die Brusthauben müssen korrekt angelegt sein, wenn die Brustpumpe eingeschaltet wird.

### DARSTELLUNG DER ERFINDUNG

Es ist deshalb eine Aufgabe der Erfindung, eine Brustpumpe zu schaffen, welche der Mutter einen optimalen und stressfreien Beginn des Abpumpens ermöglicht.

Diese Aufgabe löst eine Brustpumpe mit den Merkmalen des Patentanspruchs 1.

Die erfindungsgemässe elektrisch betriebene Brustpumpe zum Abpumpen von menschlicher Muttermilch weist ein elektrisches Pumpaggregat und eine elektronische Steuereinheit zur Steuerung des Pumpaggregats auf. Erfindungsgemäss ist das Pumpaggregat mittels der Steuereinheit zeitverzögert aktivierbar.

Dank dieser Zeitverzögerung kann die Mutter zuerst die Brustpumpe einschalten und anschliessend in Ruhe die Brusthauben auf ihre Brüste legen und dort die optimale Position suchen.

Diese Zeitverzögerung ist nicht unbedingt immer erwünscht. Routinierte Mütter oder Mütter, welche nur eine Brust abpumpen, und somit eine Hand zum Einschalten frei haben, könnten ein direktes Einschalten bevorzugen. Deshalb ist in bevorzugten Ausführungsformen wählbar, ob das Pumpaggregat beim Einschalten der Brustpumpe sofort aktiviert wird oder zeitverzögert. Vorzugsweise ist deshalb die Zeitverzögerung von einer Benützerin aktivierbar oder deaktivierbar. D.h. entweder startet die Brusthaube standardmässig sofort und nur bei individueller Aktivierung der Zeitverzögerung beginnt der Saugvorgang später. Oder die Brusthaube startet standardmässig zeitverzögert und die Mutter hat die Möglichkeit, diese Verzögerung auszuschalten.

Vorzugsweise weist die Brustpumpe einen von einer Benützerin betätigbaren Schalter zur Aktivierung der Zeitverzögerung auf. Der Begriff "Schalter" ist hier breit zu verstehen, er umfasst Kippschalter, Druckknöpfe, Drehschalter, drucksensitive Flächen und alle weiteren Formen von Betätigungsmitteln, welche manuell betätigt werden, um ein Start- oder Stoppsignal an eine Steuerung weiterzuleiten. Dies umfasst auch ein Startsignal mittels eines Spracherkennungsmoduls oder einer anderen Art von Signalgeber.

Vorzugsweise weist die Brustpumpe einen Startschalter zur Aktivierung des Pumpaggregats auf, wobei der Schalter zur Aktivierung der Zeitverzögerung derselbe Schalter ist. In anderen Ausführungsformen ist der Schalter zur Aktivierung der Zeitverzögerung ein anderer Schalter.

Vorzugsweise ist der Schalter zur Aktivierung der Zeitverzögerung an oder in einem Pumpengehäuse angeordnet, in welchem das Pumpaggregat und die Steuereinheit angeordnet sind. Dies ist eine einfache und kostengünstige Variante.

Alternativ oder zusätzlich weist die Brustpumpe ein Spracherkennungsmodul zur Aktivierung der Zeitverzögerung durch eine Benützerin auf. So kann die Mutter auch während des Anlegens der Brusthauben noch entscheiden, ob sie eine Zeitverzögerung wünscht oder nicht.

Alternativ oder zusätzlich weist die Brusthaube einen mit der Steuereinheit verbundenen Schalter zur Aktivierung der Zeitverzögerung auf. Auch dies ermöglicht es der Mutter, während des Anlegens die Zeitverzögerung zu aktivieren.

Die erwähnten Schalter und das Spracherkennungsmodul lassen sich alternativ oder zusätzlich zum Deaktivieren oder zum vorzeitigen Stoppen der Zeitverzögerung einsetzen.

Damit die Mutter weiss, wieviel Zeit ihr noch bis zum Start des Saugvorgangs bleibt, ist vorzugsweise eine Anzeigeeinheit vorhanden, welche während der Zeitverzögerung ein für eine Benützerin wahrnehmbares Signal ausgibt. Das Signal ist vorzugsweise optisch und/oder akustisch.

Vorzugsweise ist eine optische Anzeigeeinheit vorhanden, welche während der Zeitverzögerung eine Indikation gibt, wann das Pumpenaggregat startet. Beispielsweise ist sie eine sich verändernde Uhr oder ein sich verändernder Balken. Die Uhr ist beispielsweise anolog oder digital dargestellt. Vorzugsweise zählt sie rückwärts. Ein Balken wird vorzugsweise langsam kleiner oder grösser und/oder die Farben verändern sich. Die Anzeigeeinheit kann auch ein sich reduzierendes Zahleninkrement sein. In anderen Ausführungsformen ist ein akustisches Signal in immer kürzeren Abständen hörbar und/oder es nimmt in der Lautstärke zu oder ab.

Damit eine Mutter, wenn sie zum Abpumpen bereit ist, nicht warten muss, ist vorzugsweise ein Mittel vorhanden, um eine aktivierte Zeitverzögerung vorzeitig zu beenden. Das Mittel ist vorzugsweise ein Schalter, beispielsweise ein Spracherkennungsmodul. Es kann derselbe Schalter sein, welcher zur Aktivierung verwendet wurde. Es kann auch ein anderer Schalter sein.

In einfachen Ausführungsformen ist die Dauer der Zeitverzögerung vorgegeben und kann nicht verändert werden. In anderen Ausführungsformen ist es der Mutter möglich, bereits zu Beginn die Dauer zu wählen, beispielsweise durch mehrfaches Drücken des Schalters, durch Drehen, falls der Schalter ein Drehknopf ist oder durch Einstellen der Dauer über ein anderes Eingabemittel, beispielsweise über einen separaten Schalter oder Drehknopf.

Weitere Ausführungsformen sind in den abhängigen Ansprüchen angegeben.

### KURZE BESCHREIBUNG DER ZEICHNUNGEN

Eine bevorzugte Ausführungsform der Erfindung ist im Folgenden anhand der Zeichnungen beschrieben, die lediglich zur Erläuterung dienen und nicht einschränkend auszulegen sind. In den Zeichnungen zeigen:
- Figur 1: eine schematische Darstellung einer erfindungsgemässen Brustpumpe mit einem Flussdiagramm, welches die Steuerung zeigt und
- Figur 2: eine schematische Darstellung eines Gehäuses mit Inhalt der Brustpumpe gemäss Figur 1.

### BESCHREIBUNG BEVORZUGTER AUSFÜHRUNGSFORMEN

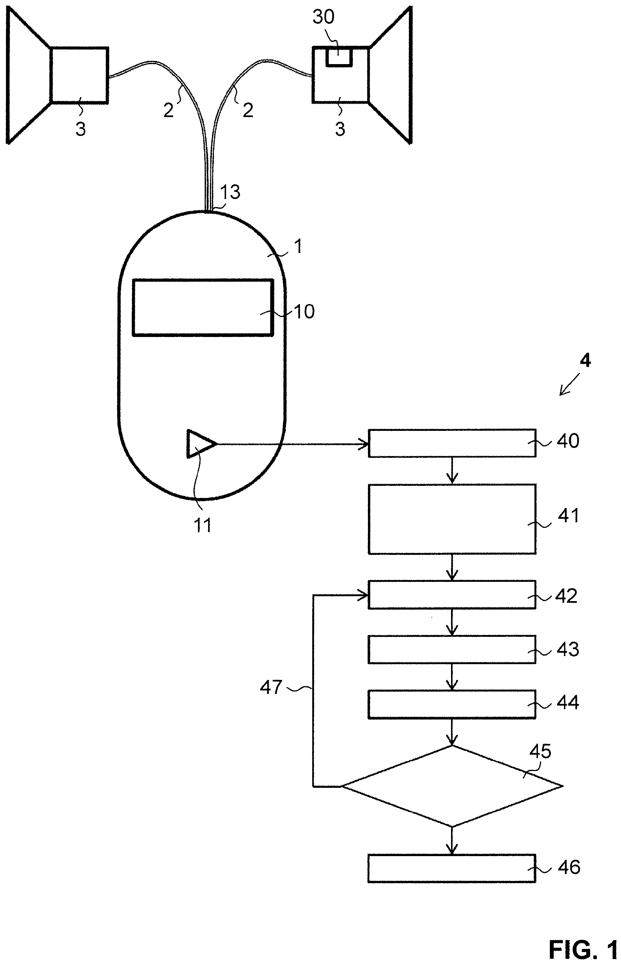

Figur 1 zeigt eine erfindungsgemässe Brustpumpe mit einem Gehäuse 1 und einem Sauganschluss 13. Im Sauganschluss 13 sind zwei Saugschläuche 2 eingesteckt, welche je zu einer Brusthaube 3 führen.

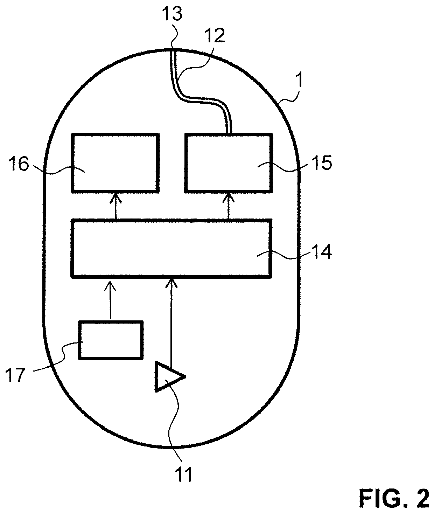

Im Gehäuse 1 sind, wie in Figur 2 erkennbar ist, ein Pumpaggregat 15 mit einem Elektromotor, eine Steuereinheit 14 zur Steuerung des Pumpaggregats 15 und eine Displaysteuerung 16 angeordnet. Vom Pumpaggregat 15 führt ein Verbindungskanal 12, üblicherweise ein Schlauch, zum Sauganschluss 13. In anderen Ausführungsformen endet das Pumpaggregat selber am Sauganschluss 13. Die Steuerungseinheit 14 verfügt über ein Zeitverzögerungsmodul, beispielsweise eine Schaltung mit einem oder mehreren Relais.

Am oder im Gehäuse 1 ist ferner eine Anzeigeeinheit, hier in Form eines Displays 10, angeordnet, wie in Figur 1 erkennbar ist. Ferner ist ein Schalter 11 vorhanden. Der Schalter 11 dient in diesem Beispiel zum Ein- und Ausschalten der Brustpumpe und gleichzeitig zur Aktivierung der Zeitverzögerung.

Anhand des Flussdiagramms ist die Wirkungsweise erkennbar. Wird der Schalter 11 von der Mutter betätigt, so startet die Pumpe sofort. Wird der Schalter 11 jedoch etwas länger gedrückt, ertönt ein kurzes akustisches Signal. Die Mutter erkennt dadurch, dass die Zeitverzögerung aktiviert wurde. Die Steuerung 14 wechselt somit in den Zeitverzögerungsmodus. Dies ist der Start 40.

Die Steuerung 14, welche mit der Displaysteuerung 16 verbunden ist oder diese mit beinhaltet, aktiviert nun den Display 10. Auf dem Display 10 erscheint eine Zahl, beispielsweise die Zahl 10. Dies ist der erste Schritt 41.

In einem zweiten Schritt 42 wartet die Steuerung 14 eine definierte Zeit, beispielsweise 1 Sekunde, und verringert dann ihr Dekrement und zeigt anschliessend auf der Anzeige eine reduzierte Zahl an, hier die Zahl 9. Die optische Anzeige zeigt somit ein sich reduzierendes Zahleninkrement an. Dies sind die dritten und vierten Schritte 43 und 44. Es wird in der Entscheidung 45 überprüft, ob die vordefinierte Dauer der Verzögerung erfüllt ist. Wenn ja, ist das Ende der Verzögerung 46 erreicht und die Steuereinheit startet das Pumpaggregat. Ist die Dauer noch nicht erreicht, so werden in einer Schlaufe 47 die zweiten bis vierten Schritte 42, 43, 44 wiederholt. Eine allfällige frühzeitige Deaktivierung der Zeitverzögerung lässt sich je nach Ausführungsform ebenfalls mittels dieses Schalters 11 durchführen.

Alternativ oder zusätzlich ist in einigen Ausführungsformen mindestens eine der Brusthauben mit einem Signalgeber 30, hier ein Schalter, ausgebildet, um je nach Ausführungsform entweder die Zeitverzögerung frühzeitig zu deaktivieren und/oder die Zeitverzögerung vor dem Pumpvorgang einzuschalten oder abzuschalten.

In anderen Ausführungsformen ist alternativ oder zusätzlich ein Spracherkennungsmodul 17 im oder am Gehäuse 1 angeordnet, welches ebenfalls mit der Steuereinheit 14 verbunden ist.

Die erfindungsgemässe Brustpumpe ermöglicht auf eine einfache Art und Weise einen stressfreien Start beim Abpumpen von Muttermilch.

### BEZUGSZEICHENLISTE

| | | | |
|---|---|---|---|
| 1 | Brustpumpe | | |
| 10 | Display | 4 | Flussdiagramm der Steuerung der Zeitverzögerung |
| 11 | Schalter | | |
| 12 | Verbindungskanal | 40 | Start Verzögerung |
| 13 | Sauganschluss | 41 | erster Schritt |
| 14 | Steuereinheit | 42 | zweiter Schritt |
| 15 | Pumpaggregat | 43 | dritter Schritt |
| 16 | Displaysteuerung | 44 | vierter Schritt |
| 17 | Spracherkennungsmodul | 45 | Entscheidung |
| | | 46 | Ende der Verzögerung |
| 2 | Saugschlauch | 47 | Schlaufe |
| | | | |
| 3 | Brusthaube | | |
| 30 | Signalgeber | | |

## Patentansprüche

1. Elektrisch betriebene Brustpumpe zum Abpumpen von menschlicher Muttermilch mit einem elektrischen Pumpaggregat (15) und einer elektronischen Steuereinheit (14) zur Steuerung des Pumpaggregats (15), **dadurch gekennzeichnet, dass** das Pumpaggregat (15) mittels der Steuereinheit (14) zeitverzögert aktivierbar ist.

2. Brustpumpe nach Anspruch 1, wobei die Zeitverzögerung von einer Benützerin aktivierbar oder deaktivierbar ist.

3. Brustpumpe nach einem der Ansprüche 1 oder 2, wobei sie einen von einer Benützerin betätigbaren Schalter (11, 30, 17) zur Aktivierung der Zeitverzögerung aufweist.

4. Brustpumpe nach Anspruch 3, wobei sie einen Startschalter (11) zur Aktivierung des Pumpaggregats aufweist und wobei der Schalter zur Aktivierung der Zeitverzögerung der Schalter zur Aktivierung des Pumpaggregats ist.

5. Brustpumpe nach Anspruch 3, wobei sie einen Startschalter (11) zur Aktivierung des Pumpaggregats aufweist und wobei der Schalter zur Aktivierung der Zeitverzögerung ein anderer Schalter ist.

6. Brustpumpe nach einem der Ansprüche 3 bis 5, wobei das Pumpaggregat (15) und die Steuereinheit (14) in einem Pumpengehäuse (1) angeordnet sind und wobei der Schalter (11, 17) zur Aktivierung der Zeitverzögerung am oder im Pumpengehäuse (1) angeordnet ist.

7. Brustpumpe nach einem der Ansprüche 1 oder 2, wobei die Brustpumpe ein Spracherkennungsmodul (17) zur Aktivierung der Zeitverzögerung durch eine Benützerin aufweist.

8. Brustpumpe nach einem der Ansprüche 1 oder 2, wobei die Brustpumpe mindestens eine Brusthaube (3) zum Anlegen an eine menschliche Mutterbrust aufweist und wobei die Brusthaube (3) einen mit der Steuereinheit (14) verbundenen Schalter (30) zur Aktivierung der Zeitverzögerung aufweist.

9. Brustpumpe nach einem der Ansprüche 1 bis 8, wobei eine Anzeigeeinheit (10) vorhanden ist, welche während der Zeitverzögerung ein für eine Benützerin wahrnehmbares Signal ausgibt.

10. Brustpumpe nach einem der Ansprüche 1 bis 9, wobei eine optische Anzeigeeinheit (10) vorhanden ist, welche während der Zeitverzögerung eine Indikation gibt, wann das Pumpenaggregat startet.

11. Brustpumpe nach Anspruch 10, wobei die optische Anzeigeeinheit (10) eine sich verändernde Uhr oder einen sich verändernden Balken oder ein sich reduzierendes Zahleninkrement anzeigt.

12. Brustpumpe nach einem der Ansprüche 9 bis 11, wobei die Anzeigeeinheit ein akustisches Signal ausgibt.

13. Brustpumpe nach einem der Ansprüche 1 bis 12, wobei ein Mittel vorhanden ist, um eine aktivierte Zeitverzögerung vorzeitig zu beenden.

14. Brustpumpe nach Anspruch 13, wobei das Mittel ein Schalter (11) ist.

15. Brustpumpe nach einem der Ansprüche 1 bis 14, wobei die Dauer der Zeitverzögerung durch die Benützerin wählbar ist.
